# FASCICULE DE BREVET EUROPEEN

(11) **EP 2 102 148 B1**
(45) Date de publication et mention de la délivrance du brevet: **16.09.2015**
(21) Numéro de dépôt: 07857856.4
(22) Date de dépôt: 19.12.2007
(51) Int. Cl.: C07C 233/05, A01N 25/02, C11D 3/43, A01P 3/00, A01P 7/04, A01P 13/00, A01P 21/00

(54) **NOUVEAUX COMPOSES BIS(DIALKYLAMIDES), PROCEDE DE PREPARATION ET UTILISATIONS**
NEUE BIS(DIALKYLAMID)VERBINDUNGEN, VERFAHREN ZU IHRER HERSTELLUNG UND ANWENDUNGEN DAVON
NOVEL BIS(DIALKYLAMIDE) COMPOUNDS, PROCESS FOR THE PREPARATION THEREOF AND USES THEREOF

(30) Priorité: 19.12.2006 FR 0611060
(43) Date de publication de la demande: 23.09.2009
(73) Titulaire: Rhodia Opérations, 93306 Aubervilliers (FR)
(72) Inventeur: MAS, Jean-Manuel, Shainghai Shanghai 201103 (CN); BRAMATI, Valerio, 20020 Arese (IT); GUGLIERI, Massimo, 75005 Paris (FR); LOURENCO, Wagner Célio Ferraz, CEP-13083-130 Sao-Paulo, SP (BR)
(74) Mandataire: Jacobson, Claude
(86) Numéro de dépôt international: PCT/EP2007/064235
(87) Numéro de publication internationale: WO 2008/074837

(56) Documents cités:
- EP-A- 0 453 899
- US-A- 3 288 794
- US-A1- 2005 003 312
- DATABASE CA [Online] CHEMICAL ABSTRACTS SERVICE, COLUMBUS, OHIO, US; NOZOE, HIROSHI ET AL: "Cellulose compositions, their films with reduced optical anisotropy and moisture permeability, modifiers for them, and polarizer protection films, liquid crystal displays, and silver halide photographic materials using them" XP002438173 extrait de STN Database accession no. 2006:465056 -& JP 2006 124649 A (FUJI PHOTO FILM CO., LTD., JAPAN) 18 mai 2006 (2006-05-18)
- GIANNELLA M ET AL: "MOLECULAR REQUIREMENTS OF THE ACTIVE SITES OF THE CHOLINERGIC RECEPTORS. XII. 3-METHYL-2-OXO-1-DIMETHYLAMINOMETHYLCYCLOP ENTANE METHIODIDE AS A NEW SELECTIVE AGONIST FOR THE NICOTINIC RECEPTOR" FARMACO, EDIZIONE SCIENTIFICA, SOCIETA CHIMICA ITALIANA, PAVIA, IT, vol. 35, no. 4, 1980, pages 253-262, XP008080206 ISSN: 0430-0920
- GAJEWSKI JOSEPH J ET AL: "Variable transition state structure in 3,3-sigmatropic shifts from .alpha.-secondary deuterium isotope effects" JOURNAL OF THE AMERICAN CHEMICAL SOCIETY, AMERICAN CHEMICAL SOCIETY, WASHINGTON, DC, US, vol. 101, no. 22, 1979, pages 6693-6704, XP002438169 ISSN: 0002-7863
- DATABASE CASREACT CHEMICAL ABSTRACT SERVICE, COLUMBUS, OHIO, US; an89:129083; 1989, MALKHASYAN, A. TS. ET AL.: "DOKLADY AKADEMII NAUK ARMYANSKOI SSR (1978), 66(3), 156-159" XP002438174
- KIM YEON CU ET AL: "Living polymerization of N,N-diphenylacrylamide with triisobutylaluminum" MACROMOLECULAR: RAPID COMMUNICATIONS, WILEY VCH VERLAG, WEINHEIM, DE, vol. 26, no. 18, 2005, pages 1499-1503, XP002438170 ISSN: 1022-1336

## Description

La présente invention a pour objet de nouveaux composés bis(dialkylamides), des utilisations de ces composés et au moins un procédé de préparation. Ces composés peuvent notamment être utilisés comme solvants, par exemple dans des formulations phytosanitaires.

L'industrie utilise de nombreux composés chimiques à titre de solvants, par exemple pour préparer des produits chimiques et des matériaux, pour formuler des composés chimiques, ou pour traiter des surfaces. Par exemple des solvants sont utilisés pour la formulation d'actifs phytosanitaires notamment sous forme de concentrés émulsionnables (Emulsifiable Concentrate "EC") destinés à être dilués dans de l'eau par l'exploitant agricole, avant application sur un champ.

L'industrie est à la recherche de nouveaux composés permettant de varier ou d'optimiser les produits et procédés dans lesquels des solvants, notamment des solvants polaires, sont à utiliser. L'industrie a notamment besoin de composés de coût modeste, présentant des propriétés d'usage intéressantes. L'industrie a également besoin de composés présentant un profile toxicologique et/ou écologique perçu comme favorable, notamment une faible volatilité (faible VOC), une bonne biodégradabilité, une faible toxicité et/ou une faible dangerosité.

On connaît l'utilisation comme solvants des dialkylamides. Il s'agit de produit de formule R-CONMe₂ ou R est un groupe hydrocarbonné comme un alkyle, typiquement en C₆-C₃₀. De tels produits sont notamment commercialisés sous la dénomination Genagen® par la société Clariant. Ces solvants trouvent des applications notamment dans le domaine phytosanitaire.

On connaît également comme solvants les diesters d'acides dicarboxyliques, notamment les diesters obtenus par estérification d'un mélange d'acide adipique, d'acide glutarique et d'acide succinique. De tels produits sont notamment commercialisés sous les dénominations Rhodiasolv® RPDE et Rhodiasovl® DIB par la société Rhodia.

Le document US 3288794 décrit des bis(dialkylamide) d'acide dicarboxyliques linéaires de formule HOOC-(CH₂)_{z}-COOH comme l'acide adipique (z=4), l'acide glutarique (z=3), l'acide succinique (z=2). Ces produits sont solides, les points de fusion sont de l'ordre de 80°C. Ils ne peuvent donc pas être utilisés comme solvants à des températures plus modestes, notamment à température ambiante.

Le document EP 186950 décrit des bis(amides) d'acides dicarboxyliques, obtenus à partir d'un monoamide, de CO et d'une amine. Ce document décrit en particulier la préparation de l'adipamide de formule H₂NOC-(CH₂)_{z}-CONH₂ où z=4. Le document enseigne l'utilisation des composés comme monomères ou intermédiaires destinés à la préparation de polymères.

Le document US 4588833 décrit la préparation d'amides d'acide succinique substitué. Ce document décrit en particulier la préparation de composés de type XOC-CH₂-CHR⁶-CONEt₂ où R⁶ est un méthyle ou un éthyle. Les produits sont préparés par mise en présence de CO, d'un alcool ou d'une amine HX, et d'acide crotonique dialkylamide ou d'acide pent-3-enoique diéthylamide. Le document enseigne l'utilisation des composés comme anti-oxydants, comme stabilisants pour plastiques, ou comme intermédiaires de synthèse organique.

Le document US 2005/0003312 décrit des composés de formule R¹R²NOC-(R)ₚ-CONR³R⁴, et en particulier les composés spécifiques suivants : (le) : avec R¹=R²=R³=R⁴=2-éthylhexyle et R= -CH₂-CH(tBu)-CH₂- et (If) : avec R¹=R²=R³=R⁴=2-éthylhexyle et R= -CH₂-CH(CH₃)-.

Le document US 3 288 794 décrit des amides solides de formule R₃R₄N-CO-R-CO-NR₁R₂, et plus particulièrement des composés où R est une chaîne linéaire et R₁=R₂=R₃=R₄=Me.

Il demeure un besoin, comme expliqué plus haut, pour d'autres composés, pouvant être utiles notamment comme solvants.

Pour répondre à ce besoin, on propose ici un composé dé formule (la) suivante :

R²R³NOC-R^{1a}-CONR⁴R⁵ (Ia)

où R², R³, R⁴ et R⁵, identiques ou différents, sont:
- des groupes alkyles, linéaires ou ramifiés ou cycliques, en C₁-C₆ de préférence en C₁-C₄, ou
- des groupes phényle,
caractérisé en ce que
R^{1a} est un groupe divalent de formule (IIa):

-CH₂-CH₂-(CHR⁷)_{z}-(CHR⁶)ₓ-(CHR⁷)_{y}- (IIa)

où:
- x est un nombre entier supérieur à 0,
- y est un nombre entier moyen supérieur ou égal à 0,
- z est un nombre entier moyen supérieur ou égal à 0,
- R⁶, identique ou différent, est un groupe alkyle en C₁-C₆ de préférence en C₁-C₄, et
- R⁷, identique ou différent, est un atome d'hydrogène ou un groupe alkyle en C₁-C₆ de préférence en C₁-C₄.

L'invention a pour objet des composés de formule (Ia) susmentionnée dans laquelle :
R², R³, R⁴ et R⁵, identiques ou différents, sont des groupes alkyles linéaires en C₁-C₄, et R^{1a} est un groupe divalent de formule (IIa) :

   -CH₂-CH₂-(CHR⁶)ₓ- (IIa)

   où :
   - x est un nombre entier supérieur à 0, et
   - R⁶, identique ou différent, est un groupe alkyle en C₁-C₆.

L'invention a également pour objet un procédé de préparation du composé de l'invention.

L'invention a également pour objet l'utilisation du composé de l'invention dans des formulations. L'invention a également pour objet un procédé de préparation des formulations par ajout du composé de l'invention. L'invention a également pour objet des formulations comprenant le composé de l'invention. Les formulations peuvent être notamment des formulations phytosanitaires.

L'invention a également pour l'utilisation du composé comme solvant, co-solvant et/ou inhibiteur de cristallisation. L'invention a également pour objet un procédé de solvatation, co-solvatation et/ou inhibition de cristallisation par ajout du composé de l'invention.

### Composé de l'invention

On décrit ici des composés de formule (la) suivante:

R²R³NOC-R^{1a}-CONR⁴R⁵ (Ia)

où R², R³, R⁴ et R⁵, identiques ou différents, sont des groupes alkyles, linéaires ou ramifiés, en C₁-C₆ de préférence en C₁-C₄,
caractérisé en ce que
R^{1a} est un groupe divalent de formule (IIa):

-CH₂-CH₂-(CHR⁷)_{z}-(CHR⁶)ₓ-(CHR⁷)_{y}- (IIa)

où:
- x est un nombre entier supérieur à 0,
- y est un nombre entier moyen supérieur ou égal à 0,
- z est un nombre entier moyen supérieur ou égal à 0,
- R⁶, identique ou différent, est un groupe alkyle en C₁-C₆ de préférence en C₁-C₄, et
- R⁷, identique ou différent, est un atome d'hydrogène ou un groupe alkyle en C₁-C₆ de préférence en C₁-C₄.

Les groupes R², R³, R⁴ et R⁵, identiques ou différents, sont de préférence choisis parmi les groupes méthyle, éthyle, propyle (n-propyl), isopropyle, n-butyle, isobutyle, n-pentyle, amyle, isoamyle, hexyle, cyclohexyle. Ils sont de préférence identiques.

Les groupes R⁷ peuvent notamment être linéaires, ramifiés ou cycliques:

Selon un mode particulier le groupe R^{1a} est de préférence un groupe tel que y = z = 0.

Les composés de l'invention sont des composés de formule (la) telle que définie ci-dessus, dans laquelle R², R³, R⁴ et R⁵, identiques ou différents, sont choisis parmi les groupes méthyle, ethyle, n-propyle et n-butyle.

Le groupe R^{1a} est de préférence un groupe tel que:
- x=1,
- y = z - 0,
- R⁶ = méthyle.

Un tel groupe R^{1a} est tel que le composé de formule HOOC-R^{1a}-COOH est l'acide 2-méthylglutarique.

De préférence, pour le composé:
- le groupe R^{1a} est tel que:
   - x= 1,
   - y = z = 0,
   - R⁶ = méthyle, et
- R², R³, R⁴ et R⁵ sont identiques et choisis parmi les groupes méthyle, éthyle ou n-propyl.

Des exemples de composés sont les composés de formule suivante:
- 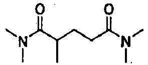 (R⁶ = méthyle; x=1, y=z=0; R²=R³=R⁴=R⁵= méthyle)
- 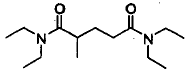 (R⁶ = méthyle; x=1, y=z=0; R²=R³=R⁴=R⁵= éthyle),
- 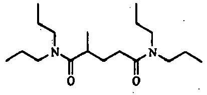 (R⁶ = méthyle; x=1, y=z=0; R²=R³=R⁴=R⁵= n-propyle).

Le composé peut notamment être un produit d'amidification ou de trans-amidification. Il peut notamment être compris dans un mélange de composés, par exemple dans un produit de réaction comprenant plusieurs composés différents.

Le composé de l'invention présente avantageusement une température de fusion inférieure ou égale à 25°C.

On mentionne que le composé est typiquement différent d'un composé de formule (phényl)₂-NOC-CH₂-CH₂-CH(CH₃)-CON-(phényl)₂.

### Composition de matière comprenant le composé de l'invention

Le composé de l'invention peut être compris dans une composition de matière. Par composition de matière, on entend une composition, plus ou moins complexe, comprenant plusieurs composés chimiques. Il peut s'agir typiquement d'un produit de réaction non purifié ou modestement purifié. Le composé de l'invention pourra notamment être isolé et/ou commercialisé et/ou utilisé sous forme d'une composition de matière le comprenant.

Dans la composition de matière le composé de l'invention peut représenter au moins 10% en poids. De préférence, il s'agit du composé principal de la composition de matière. Par composé principal, on entend dans la présente demande, le composé dont la teneur est la plus élevée, même si sa teneur est inférieure à 50% en poids (par exemple dans un mélange de 40% de A, de 30% de B, et de 30% de C, le produit A est le composé principal). Encore plus préférablement le composé de l'invention représente au moins 50% en poids de la composition de matière, par exemple de 70 à 95% en poids, et même de 75 à 90% en poids.

Comme indiqué plus haut, la composition de matière peut être un produit de réaction, notamment un produit d'amidification ou de trans-amidification.

La composition de matière peut notamment comprendre en plus du composé de l'invention, un produit de formule (Ib) suivante:

R²R³NOC-R^{1b}-CONR⁴R⁵ (Ib)

où:
- R², R³, R⁴ et R⁵, identiques ou différents, sont tels que définis ci-dessus,
- R^{1b} est un groupe divalent de formule (IIb):

   -CH₂-(CHR⁷)_{z}-(CHR⁶)ₓ-(CHR⁷)_{y}- (IIb)

   où:
   - x est un nombre entier supérieur à 0,
   - y est un nombre entier moyen supérieur ou égal à 0,
   - z est un nombre entier moyen supérieur ou égal à 0,
   - R⁶, identique ou différent, est un groupe alkyle en C₁-C₆ de préférence en C₁-C₄, et
   - R⁷, identique ou différent, est un atome d'hydrogène ou un groupe alkyle en C₁-C₆ de préférence en C₁-C₄.

Le groupe R^{1b} est de préférence un groupe tel que:
- x= 1,
- y = Z = 0,
- R⁶ = éthyle.

Un tel groupe R^{1b} est tel que le composé de formule HOOC-R^{1b}-COOH est l'acide 2-éthylsuccinique.

La composition de matière peut notamment comprendre en plus du composé de l'invention, un produit de formule (Ic) suivante:

R²R³NOC-(CH₂)_{w}-CONR⁴R⁵ (Ic)

où:
- R², R³, R⁴ et R⁵, identiques ou différents, sont tels que définis ci-dessus, et
- w est un nombre entier supérieur à 0, de préférence égal à 4.
Quand w=4, le produit de forme HOOC-(CH₂)₄-COOH est l'acide adipique.

La composition de matière est notamment être un produit d'amidification ou de trans-amidification d'un mélange d'acide méthylglutarique, d'acide éthylsuccinique et éventuellement d'acide adipique, où d'un diester de ce mélange. Dans ce cas elle comprendra un mélange comprenant:
- le composé de l'invention dans lequel R^{1a} est tel que le composé de formule HOOC-R^{1a}-COOH est l'acide 2-méthylglutarique,
- le produit de formule (Ib) dans lequel R^{1b} est tel que le composé de formule HOOC-R^{1b}-COOH est l'acide 2-éthylsuccinique, et
- éventuellement le produit de formule (Ic) dans lequel w=4.

La teneur en produit de formule (Ib) dans la composition de matière peut par exemple être de 5 à 30% en poids, ou même de 5 à 20%.

La teneur en produit de formule (Ic) dans la composition de matière peut par exemple être de nulle ou inférieure à 15%, ou même inférieure à 10%.

Il n'est pas exclu que la composition de matière comprenne d'autres produits que ceux identifiés ci-dessus. In peut s'agir notamment d'espèces non converties en amides, ou de produits amides différents des composés de l'invention et des produits par exemple des diamides de l'acide adipique de formule (R²R³NOC-(CH₂)₄-CONR⁴R⁵). Il peut s'agir également de produits partiellement amidifiés ou trans-amidifiés (où une des fonctions acides ou esters a été convertie en amide, sur deux disponibles).

Une composition de matière particulièrement utile et/ou pratique et/ou simple d'accès comprend :
- de 70 à 95 % en poids du composé de formule (la), de préférence de 75 à 90%, de préférence de 79 à 86%, de préférence du composé de formule 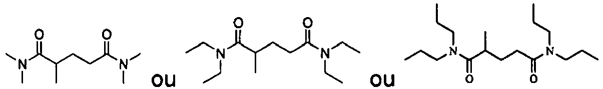
- de 5 à 30 % en poids d'un composé de formule (Ib), de préférence de 5 à 20%, de préférence de 7 à 12%, de préférence de N,N,N,N-tetraméthyle 2-éthylsuccinamide ou de N,N,N,N-tetraéthyle 2-éthylsuccinamide ou N,N,N,N-tetra(n-propile) 2-éthylsuccinamide,
- éventuellement, au plus 15% en poids d'un composé de formule (Ic), de préférence au plus 10%, de préférence au plus 8% d'un autre composé, par exemple de 2 à 7%, de préférence de N,N,N,N-tetraméthyladipamide ou N,N,N,N-tetraéthyladipamide, ou N,N,N,N-tetra(n-propyl)adipamide.

Le total des pourcentages, avec le cas échéant d'autres produits pouvant être présents, doit être de 100%.

La composition de matière présente avantageusement une température de fusion inférieure ou égale à 25°C.

### Procédé de préparation du composé ou de la composition de matière

Le composé de l'invention peut être préparé par toute méthode appropriée. On préférera notamment mettre en oeuvre des réactions d'amidification ou de trans-amidification sur des diacides ou des diesters analogues. De telles réactions sont connues de l'homme du métier. Ces méthodes peuvent être mises en oeuvre de manière similaire pour des compositions de matières. On pourra notamment mettre en oeuvre des réactions d'amidification ou de trans-amidification sur des mélanges de diacides ou de diesters analogues.

Ainsi un procédé de préparation d'un composé de l'invention, le cas échéant dans une composition de matière, comprend une étape d'amidification ou de trans-amidification par un composé de formule R²R³NH et/ou HNR⁴R⁵ d'un composé de formule (l'a) suivante:

R⁸OOC-R^{1a}-COOR⁸ (I'a)

le composé de formule (l'a) étant le cas échéant en mélange avec un composé de formule (I'b) suivante:

R⁸OOC-R^{1b}-COOR⁸ (I'b),

et le cas échéant en mélange avec un composé de formule (I'c) suivante:

R⁸OOC-(CH₂)_{w}-COOR⁸ (I'c)

où
- R⁸ est un atome d'hydrogène ou un alkyle en C₁-C₆ de préférence un méthyle, et
- R^{1a} est tel que défini plus haut,
- R^{1b} est tel que défini plus haut, et
- w est tel que défini plus haut.

Les composés de formules (I'à), (I'b), et (I'c) sont considérés comme des diacides ou diesters analogues respectivement du composé de l'invention, du produit de formule (Ib), et du produit de formule (Ic).

La réaction d'amidification ou de trans-amidification peut être menée de manière continue, semi-continue, ou discontinue ("batch"). Afin d'améliorer le taux de conversion de la réaction (taux de conversion de l'acide ou du diesters) et/ou de diminuer la quantité de produit non réagi dans une composition de matière et/ou d'augmenter la productivité, on peut notamment éliminer des sous produits de réaction au cours de cette dernière. On peut par exemple éliminer par évaporation ("stripping") des alcools formés au cours d'une réaction de trans-amidification. On note qu'on peut utiliser un très large excès d'amine, un sel d'amine ou tout autre moyen connu de l'homme du métier, par exemple décrit dans l'ouvrage "March's Advanced Organic Chemistry" de Michael B. Smith et Jerry March, 5e édition, John Wiley & Sons, pages 506-511.

La réaction peut être suivie d'étapes de filtration et/ou de purification par exemple par distillation.

On note que la réaction d'amidification ou de trans-amidification peut transiter par des intermédiaires activés tels les chlorures d'acides obtenus, par exemple, à partir des composés de formules (l'a) et (I'b) par réaction avec le chlorure de thionyle. La séparation de l'acide chlorhydrique, sous-produit au cours de ce type de réaction d'amidification, du milieu réactionnel par tout moyen adapté (formation de sel, distillation), constitue un moteur pour déplacer l'équilibre réactionnel vers la formation de l'amide recherché.

Les diacides ou diesters, sous forme de mélanges pour l'obtention d'une composition de matière, peuvent notamment être obtenus à partir d'un mélange de composés dinitriles notamment produit et récupéré dans le procédé de fabrication de l'adiponitrile par double hydrocyanation du butadiène. Ce procédé utilisé à grande échelle dans l'industrie pour produire la grande majorité de l'adiponitrile consommé dans le monde est décrit dans de nombreux brevets et ouvrages.

La réaction d'hydrocyanation du butadiène conduit majoritairement à la formation de dinitriles linéaires mais également à une formation de dinitriles ramifiés dont les deux principaux sont le méthylglutaronitrile et l'éthylsuccinonitrile.

Dans les étapes de séparation et de purification de l'adiponitrile, les composés dinitriles ramifiés sont séparés par distillation et récupérés, par exemple, comme fraction de tête dans une colonne de distillation.

Les dinitriles ramifiés peuvent par la suite être transformés en diacides ou en diesters. Un des procédés possibles pour la transformation des dinitriles en diesters correspond à la mise en oeuvre de la réaction de PINNER, notamment décrite dans le brevet français n° 1488857. Sommairement, ce procédé consiste à faire réagir les composés dinitriles avec un alcool en présence d'un acide minéral fort tel que l'acide suflurique, puis à hydrolyser les produits obtenus pour récupérer des diesters par distillation. Ce document décrit également un mode de réalisation particulier du procédé qui consiste à faire passer le mélange de composés dinitriles et l'alcool dans un bain de sels fondus à base de différents sulfates alcalins et ammonium pour éviter la formation de sulfate d'ammonium et récupérer l'ammoniaque par extraction à la vapeur d'eau.

Des diesters utiles peuvent également être obtenus par réaction entre les composés dinitriles, de l'eau, et un alcool en phase gaz et en présence d'un catalyseur solide. La température de réaction est avantageusement supérieure à la température de condensation des diesters formés. Comme catalyseur, on peut utiliser un catalyseur solide acide tel que par exemple un gel de silice, un mélange silice-alumine, des acides boriques ou phosphoriques supportés. On peut également utiliser des alumines macroporeuses telles que celles décrites dans le document EP805801.

La température de réaction de transformation de dinitriles en diesters peut être comprise entre 200°C et 450°C, de préférence entre 230°C et 350°C. La réaction peut être réalisée sous une pression quelconque, avantageusement comprise entre 0,1 et 20 bar. En sortie de réacteur les vapeurs peuvent être refroidies rapidement à une température inférieure ou égale à 150°C. Du mélange obtenu, on peut séparer par distillation l'ammoniac, puis l'eau et l'alcool en excès.

Des diesters utiles peuvent également être obtenus par réaction entre les composés dinitriles et une base minérale, pour obtenir des sels d'acide, puis neutralisation de ces sels par un acide, suivie d'une estérification avec un alcool. Un procédé utile est notamment détaillé dans la demande de brevet français déposée le 9 juin 2006 sous le n° 06 05119.

Des diacides utiles peuvent être obtenus par réaction entre les composés dinitriles et une base minérale, pour obtenir des sels d'acide, puis neutralisation de ces sels par un acide. Des diacides utiles peuvent également être obtenus par hydrolyse acide des composés dinitriles.

### Utilisations

Le composé de l'invention et/ou une composition de matière le comprenant décrite ci-dessus, peut notamment être utilisé comme solvant, co-solvant et/ou inhibiteur de cristallisation, ou comme agent de coalescence.

Par co-solvant, on entend que d'autres solvants peuvent lui être associés. L'utilisation à titre de solvant ou de co-solvant comprend notamment des utilisation pour dissoudre un composé dans une formulation, dans un milieu réactionnel, l'utilisation pour solubiliser totalement ou partiellement un produit à éliminer (dégraissage, décapage), et/ou pour faciliter de décollage de films de matières. Le produit à éliminer peut notamment être une huile, des graisses, des cires, du pétrole, des résines, de la peinture, des graffitis. Il peut être utilisé comme agent de traitement préalable facilitant la suppression de graffitis après leur apparition.

Le composé de l'invention et/ou une composition de matière le comprenant décrite ci-dessus, peut notamment être utilisé, pour les fonctions indiquées ci-dessus ou pour d'autres, dans une formulation phytosanitaire, dans une formulation du nettoyage, dans une formulation de décapage, dans une formulation de dégraissage, dans une formulation de lubrifiants, dans une formulation de nettoyage ou de dégraissage textiles, dans une formulation de revêtement, par exemple dans une formulation de peinture, dans une formulation de pigments ou encre, dans une formulation plastique.

Le composé peut par exemple être utilisé à titre d'agent de coalescence dans une formulation de peinture aqueuse. Il peut être utilisé comme solvant dans une formulation de peinture non aqueuse.

Le composé peut notamment être utilisé comme agent de dégraissage sur des surfaces métalliques, par exemple des surfaces d'outils, d'objets manufacturés, de tôles, de moules, notamment en acier ou en aluminium ou en alliages de ces métaux.

Le composé peut notamment être utilisé comme solvant de nettoyage sur des surfaces dures ou des surfaces textiles. Il peut être utilisé pour nettoyage des sites industriels, par exemple des sites d'exploitation de pétrole ou de gaz, par exemple des plateformes pétrolières en mer ou non.

Le composé peut notamment être utilisé comme solvant de décapage de peinture ou de résines, sur des surfaces d'outils, par exemple des moules de fonderie, sur des surfaces des sites industriels (sols, cloisons etc...).

Le composé peut notamment être utile comme solvant de nettoyage ou de décapage de d'outils d'impression.

Les formulations de nettoyage et/ou de dégraissage peuvent notamment être des formulations pour les soins ménagers, opérés dans les foyers ou dans les domaines publiques (hôtels, bureaux, usines....). Il peut s'agir de formulation pour le nettoyage des surfaces dures comme les sols, les surfaces d'ameublement et d'équipement des cuisines et salles de bain, la vaisselle. Ces formulations peuvent également être utilisées dans la sphère industrielle pour dégraisser des produits manufacturés et/ou les nettoyés.

Le composé de l'invention et/ou une composition de matière le comprenant décrite ci-dessus, peut notamment être utilisé dans des formulations phytosanitaires comprenant un produit actif solide. Plus de détails sont donnés ci-dessous, où le mot "solvant" peut désigner le composé de l'invention ou une composition de matière le comprenant, décrite ci-dessus.

### Utilisation détaillée dans le cadre de formulations phytosanitaires

La formulation phytosanitaire est généralement une formulation phytosanitaire concentrée comprenant un composé actif.

L'agriculture utilise de nombreuses matières actives telles que des fertilisants ou des pesticides, par exemple des insecticides, herbicides ou fongicides. On parle de produits phytosanitaires actifs (ou de matière active). Les produits phytosanitaires actifs sont généralement produits sous forme pure ou très concentrée. Ils doivent être utilisés sur les exploitations agricoles à de faibles concentrations. A cette fin, ils sont généralement formulés avec d'autres ingrédients afin de permettre une dilution en poids aisée par l'exploitant agricole. On parle de formulations phytosanitaires. La dilution opérée par l'exploitant agricole est généralement réalisée par mélange de la formulation phytosanitaire avec de l'eau.

Ainsi les formulations phytosanitaires doivent permettre une dilution en poids aisée par l'exploitant agricole, afin d'obtenir un produit dans lequel le produit phytosanitaire est correctement dispersé, par exemple sous forme de solution, d'émulsion, de suspension, ou de suspo-émulsion. Les formulations phytosanitaires permettent ainsi le transport d'un produit phytosanitaire sous forme relativement concentrée, un conditionnement aisé et/ou une manipulation aisée pour l'utilisateur final. Différents types de formulations phytosanitaires peuvent être utilisés selon les différents produits phytosanitaires. On cite par exemple les concentrés émulsionnables (Emulsifiable Concentrates «EC»), les émulsions concentrées (Emulsion in water "EW"), les microémulsions ("ME"), les poudres mouillables (Wettable Powders «WP»), les granulés dispersables dans l'eau (Water Dispersible Granules, «WDG»). Les formulations qu'il est possible d'utiliser dépendent de la forme physique du produit phytosanitaire (par exemple solide ou liquide), et de ses propriétés physico-chimiques en présence d'autres composés comme l'eau ou les solvants.

Après dilution en poids par l'exploitant agricole, par exemple par mélange avec de l'eau, le produit phytosanitaire peut se trouver sous différentes formes physiques: solution, dispersion de particules solides, dispersion de gouttelettes du produit, gouttelettes de solvant dans lequel le produit est dissous... Les formulations phytosanitaires comprennent généralement des composés permettant d'obtenir ces formes physiques. Il peut par exemple s'agir de tensioactifs, de solvants, de supports minéraux, et/ou de dispersants. Bien souvent ces composés n'ont pas un caractère actif, mais un caractère d'intermédiaire d'aide à la formulation. Les formulations phytosanitaires peuvent notamment être sous forme liquide, ou sous forme solide.

Afin de préparer des formulations phytosanitaires de produits phytosanitaires actifs solides, il est connu de solubiliser le produit dans un solvant. La formulation phytosanitaire comprend ainsi une solution du produit dans le solvant. La formulation peut être sous forme solide, par exemple sous forme de poudre mouillable (WP) où la solution imbibe un support inorganique, par exemple du kaolin et/ou de la silice. La formulation peut alternativement être sous forme liquide, par exemple sous forme de concentré émulsionnable (EC) présentant une seule phase liquide limpide comprenant le solvant et le produit en solution, pouvant former une émulsion par ajout d'eau, sans agitation ou avec une faible agitation. Elle peut aussi être ou sous forme d'une émulsion concentrée (EW), trouble, dont la phase dispersée dans l'eau comprend le solvant et le produit en solution dans le solvant. Elle peut aussi être forme d'une microémulsion (ME), limpide, dont la phase dispersée dans l'eau comprend le solvant et le produit en solution dans le solvant.

Certains actifs phytosanitaires solides sont souvent difficiles à formuler. Par exemple le tebuconazole est un fongicide particulièrement efficace, et d'utilisation répandue, pour la culture du soja notamment. Pour certains actifs phytosanitaires, il est difficile de réaliser des formulations concentrées, faciles à diluer pour l'exploitant agricole, stables, et sans inconvénients (avérés ou perçus) substantiels en matière de sécurité, de toxicité et/ou d'eco-toxicité. Pour certains actifs, il est difficile de formuler à des concentrations relativement élevées, avec une stabilité suffisante. En particulier il est nécessaire d'éviter l'apparition de cristaux en particulier à basse température et/ou lors de la dilution et/ou lors du stockage à température élevée de la composition diluée. Les cristaux peuvent avoir des effets négatifs, notamment boucher les filtres des dispositifs utiliser pour répandre la composition diluée, boucher les dispositifs de pulvérisation, diminuer l'activité globale de la formulation, créer des problèmes inutiles de filières de déchets pour éliminer les cristaux, et/ou provoquer une mauvaise répartition du produit actif sur le champ agricole.

Les formulations comprenant le solvant présentent notamment:
- une solubilisation de quantités importantes d'actifs,
- une absence de cristallisation, même des conditions exigeantes,
- une bonne activité biologique pouvant être due à une bonne solvatation, et/ou
- un profile de sécurité, toxicologie et/ou eco-toxicologie perçu comme favorable.

La formulation phytosanitaire peut en outre être une formulation phytosanitaire concentrée comprenant:
a) un produit phytosanitaire actif,
b) le solvant
c) éventuellement au moins agent émulsifiant, de préférence un tensioactif, et
d) éventuellement de l'eau.

### Produit phytosanitaire actif a)

Des produits phytosanitaires actifs, notamment des produits non solubles dans l'eau et solides sont connus de l'homme du métier. Le produit phytosanitaire actif peut notamment être un herbicide, un insecticide, un acaricide, un fongicide, ou un agent d'élimination des rongeurs ("rodenticide" en anglais) par exemple un raticide.

A titre d'exemples non limitatifs de matières actives convenables, on peut citer entre autres l'Amétryne, le Diuron, le Linuron, le Chlortoluron, l'Isoproturon, le Nicosulfuron, le Metamitron, le Diazinon, l'Aclonifen, l'Atrazine, le Chlorothalonil, le Bromoxynil, le Bromoxynil heptanoate, le Bromoxynil octanoate, le Mancozeb, la Manèbe, le Zineb, la Phenmédipham, le Propanyl, la série des phénoxyphénoxy, la série des hétéroaryloxyphénoxy, le CMPP, le MCPA, le 2,4-D, la Simazine, les produits actifs de la série des imidazolinones, la famille des organophosphorés, avec notamment l'Azinphos-éthyl, l'Azinphos-méthyl, l'Alachlore, le Chlorpyriphos, le Diclofop-méthyl, le Fénoxaprop-p-éthyl, le Méthoxychlore, la Cyperméthrine, le Fenoxycarbe, le cymoxanil, le chlorothalonyl, Ikes insecticides neonicotinoides, la famille des fongicide triazoles tels que l'azaconazole, bromuconazole, cyproconazole, difenoconazole, diniconazole, epoxyconazole, fenbuconazole, flusilazole, myclobutanyl, tebuconazole, triadimefon, triadimenol, des strobiturines telles que la pyraclostrobine, la picoxystrobine, l'azoxystrobine, la famoxadone, le kresoxym-methyl et la trifloxystrobine, les solfonylurées telles que le bensulfuron-methyl, le chlorimuron-ethyl, le chlorsulfuron, le metsulfuron-methyl, le nicosulfuron, le sulfomethuron-methyl, le triasulfuron, le tribenuron-methyl.

On choisi parmi cette liste les produits non-hydrosolubles.

Le produit phytosanitaire actif peut en particulier être choisi parmi les azoles, de préférence les triazoles, de préférence le tebuconazole. Le tebuconazole est la dénomination usuelle d'un composé connu de l'homme du métier, dont la formule est la suivante: 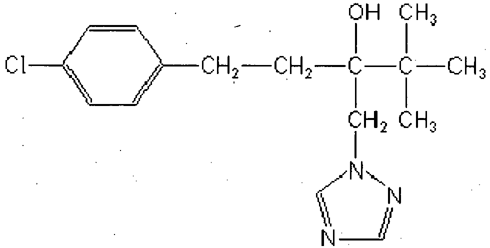 Le tebuconazle est un produit phytosanitaire solide.
A titre de triazoles différents du tebuconazole, on peut notamment citer les composés suivants: Azaconazole; bitertanol; bromuconazole; cyproconazole; diclobutrazol; difenoconazole; diniconazole; diniconazole-M; epoxiconazole; etaconazole; fenbuconazole; fluotrimazole; fluquinconazole; flusilazole; flutriafol; furconazole; furconazole-cis; hexaconazole; imibenconazole; ipconazole; metconazole; myclobutanil; penconazole; prochloraz, propiconazole; prothioconazole; quinconazole; strobulurin et analogues, simeconazole; tetraconazole; triadimefon; triadimenol; triazbutil; triflumizole, triticonazole; uniconazole; uniconazole-P.

Le produit phyosanitaire actif peut en particulier être choisi parmi les dinitroanilines, comme le pendimethalin ou le trifluralin.

On peut notamment mettre en oeuvre les produits phytosanitaires actifs suivants:

| | |
|---|---|
| Alach lor | 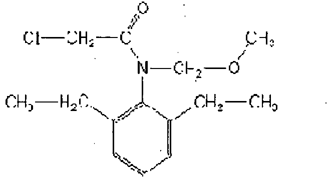 |
| Chlorpyrifos | 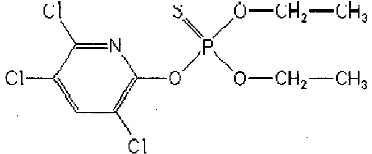 |
| alphacyperméthrine | 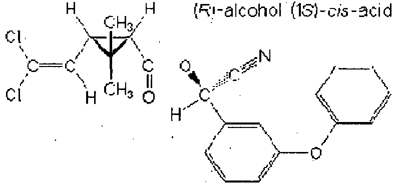 |
| | 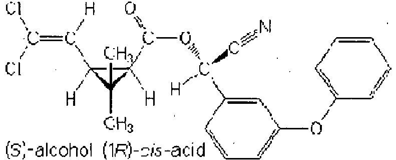 |
| | En mélange racémique et/ou en stéréoisomères isolés. |
| Phenmedipham | 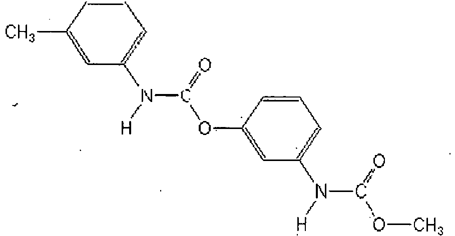 |
| Propanil | 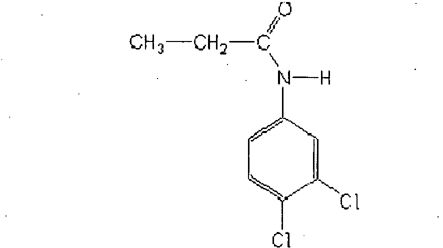 |
| Pendimethalin | 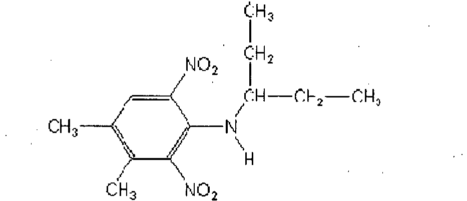 |
| triadimenol | 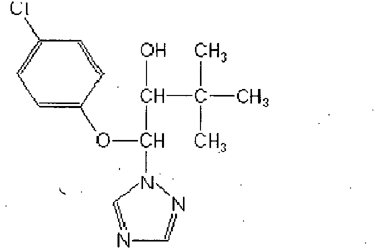 |
| Trifluralin | 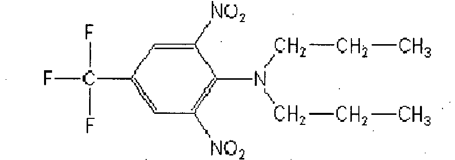 |
| Oxyfluorfen | 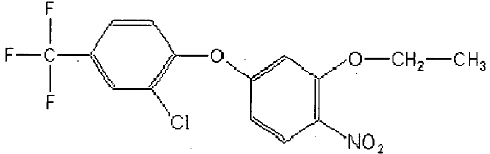 |
| Dimethoate | 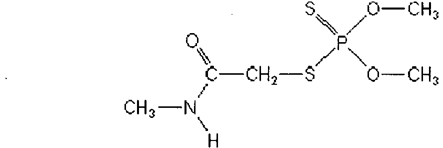 |
| Imidacloprid | 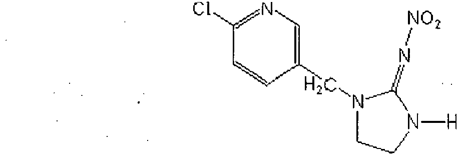 |
| Proxopur | 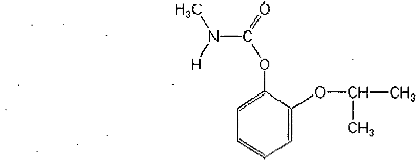 |
| Benomyl | 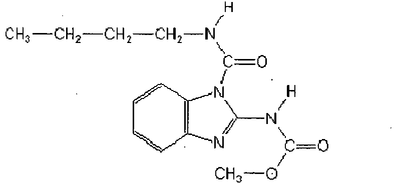 |
| Deltamethrine | 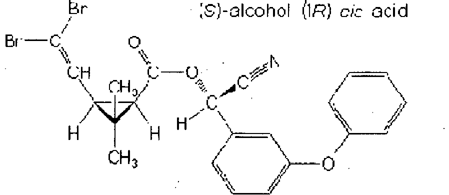 |
| Fenvalerate | 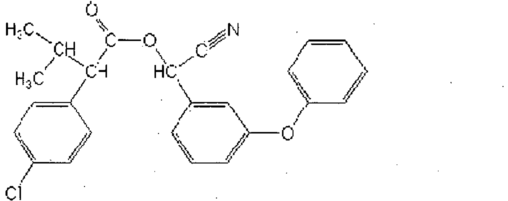 |
| Abamectin | 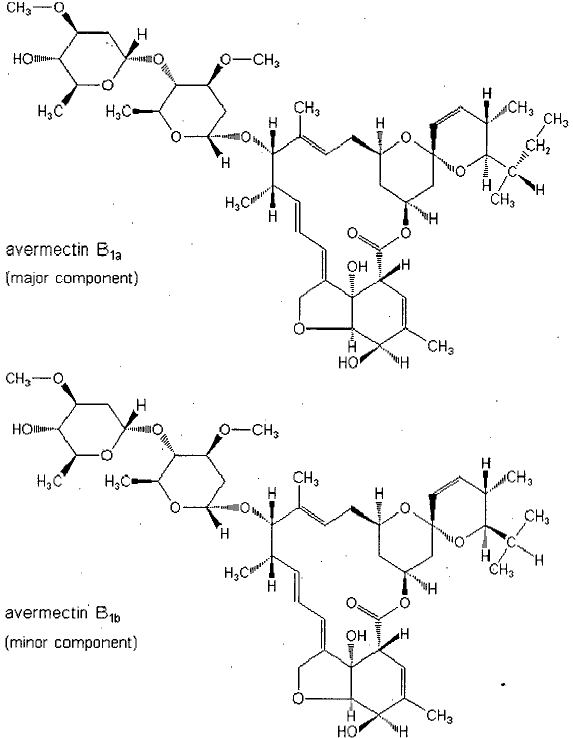 |
| Amicarbazone | 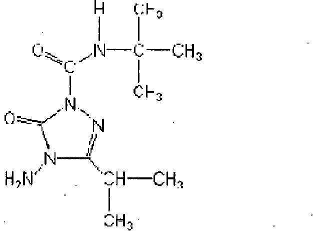 |
| Bifenthrin | 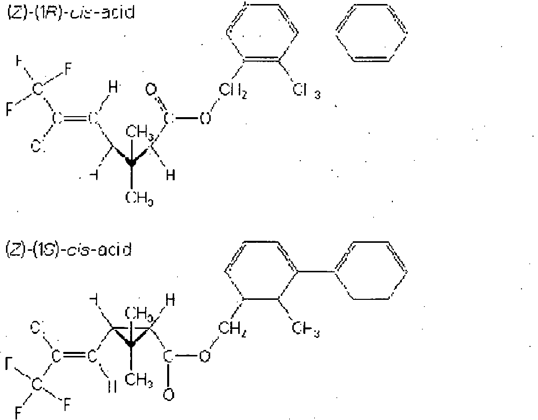 |
| Carbosulfan | 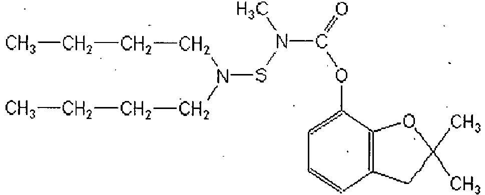 |
| Cyfluthrin | 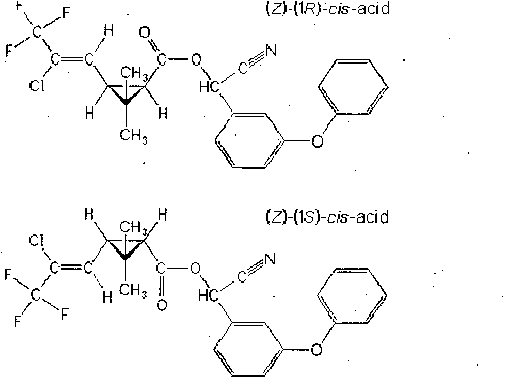 |
| Difenconazole | 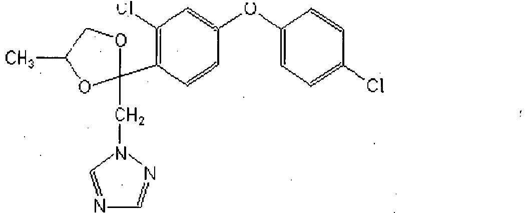 |
| Ethofenprox | 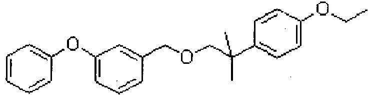 |
| Fenoxapropethyl | 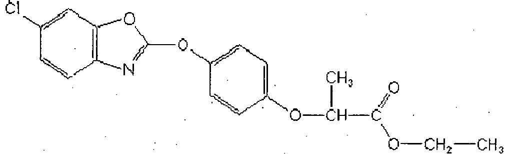 |
| Fipronil | 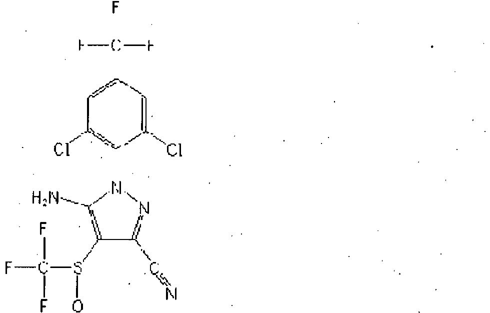 |
| Fenvalerate | 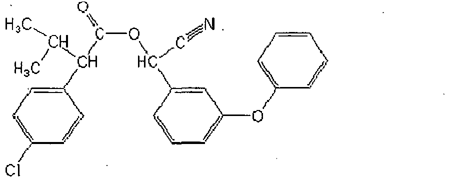 |
| Fluazifop-p-butyl | 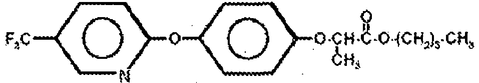 |
| Flufenouron | 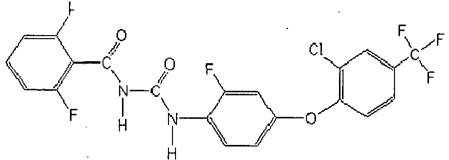 |
| Hexazinone | 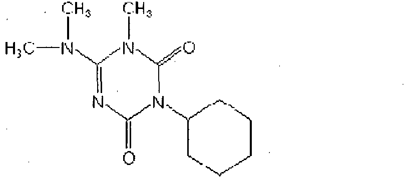 |
| Lambdacyalothrin | 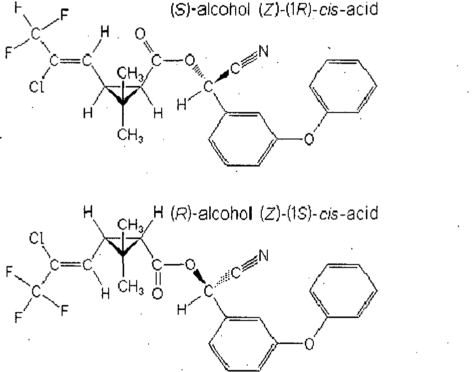 |
| Methomyl | 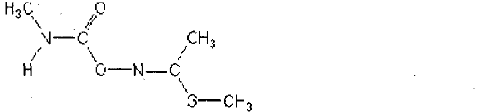 |
| Permethrin | 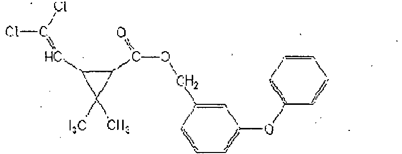 |
| Prochloraz | 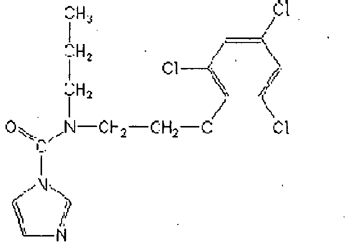 |
| Propiconazole | 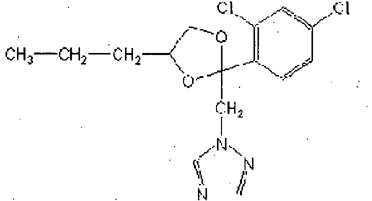 |
| Tebuconazole | 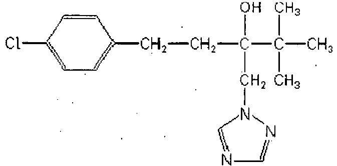 |

Ces produits et dénominations sont connus de l'homme du métier. On peut associer plusieurs produits phytosanitaires actifs.

### Agent émulsifiant c)

La formulation phytosanitaire peut comprendre un agent émulsifiant, typiquement et de préférence un tensioactif. Les agents émulsifiants sont des agents destinés à faciliter la mise en émulsion ou la dispersion après mise en présence de la formulation avec de l'eau, et/ou à stabiliser (dans le temps et/ou en température) l'émulsion ou la dispersion, par exemple en évitant une sédimentation.

Les tensioactifs sont des composés connus, qui présentent une masse molaire généralement relativement faible, par exemple inférieure à 1000 g/mol. Le tensioactif peut être un tensioactif anionique sous forme salifiée ou acide, non ionique de préférence polyalcoxylé, cationique, amphotère (terme incluant aussi les tensioactifs zwitterioniques). Il peut s'agir d'un mélange ou d'une association de ces tensioactifs.

A titre d'exemples de tensioactifs anioniques, on peut citer, sans intention de s'y limiter:
- les acides alkylsulfoniques, les acides arylsulfoniques, éventuellement substitués par un ou plusieurs groupements hydrocarbonés, et dont la fonction acide est partiellement ou totalement salifiée, comme les acides alkylsulfoniques en C₈-C₅₀, plus particulièrement en C₈-C₃₀, de préférence en C₁₀-C₂₂, les acides benzènesulfoniques, les acides naphtalènesulfoniques, substitués par un à trois groupements alkyles en C₁-C₃₀, de préférence en C₄-C₁₆, et/ou alcényles en C₂-C₃₀, de préférence en C₄-C₁₆.
- les mono- ou diesters d'acides alkylsulfosucciniques, dont la partie alkyle, linéaire ou ramifiée, éventuellement substituée par un ou plusieurs groupements hydroxylés et/ou alcoxylés, linéaires ou ramifiés en C₂-C₄ (de préférence éthoxylés, propoxylés, éthopropoxylés).
- les esters phosphates choisis plus particulièrement parmi ceux comprenant au moins un groupement hydrocarboné saturé, insaturé ou aromatique, linéaire ou ramifié, comprenant 8 à 40 atomes de carbone, de préférence 10 à 30, éventuellement substitués par au moins un groupement alcoxylé (éthoxylé, propoxylé, éthopropoxylé). En outre, ils comprennent au moins un groupe ester phosphate, mono- ou diestérifié de telle sorte que l'on puisse avoir un ou deux groupes acides libres ou partiellement ou totalement salifiés. Les esters phosphates préférés sont du type des mono- et diesters de l'acide phosphorique et de mono-, di- ou tristyrylphénol alcoxylé (éthoxylé et/ou propoxylé), ou de mono-, di- ou trialkylphénol alcoxylé (éthoxylé et/ou propoxylé), éventuellement substitué par un à quatre groupements alkyles ; de l'acide phosphorique et d'un alcool en C₈-C₃₀, de préférence en C₁₀-C₂₂ alcoxylé (éthoxylé ou éthopropoxylé); de l'acide phosphorique et d'un alcool en C₈-C₂₂, de préférence en C₁₀-C₂₂, non alcoxylé.
- les esters sulfates obtenus à partir d'alcools saturés, ou aromatiques, éventuellement substitués par un ou plusieurs groupements alcoxylés (éthoxylés, propoxylés, éthopropoxylés), et pour lesquels les fonctions sulfates se présentent sous la forme acide libre, ou partiellement ou totalement neutralisées. A titre d'exemple, on peut citer les esters sulfates obtenus plus particulièrement à partir d'alcools en C₈-C₂₀, saturés ou insaturés, pouvant comprendre 1 à 8 motifs alcoxylés (éthoxylés, propoxylés, éthopropoxylés) ; les esters sulfates obtenus à partir de phénol polyalcoxylé, substitués par 1 à 3 groupements hydroxycarbonés en C₂-C₃₀, saturés ou insaturés, et dans lesquels le nombre de motifs alcoxylés est compris entre 2 et 40 ; les esters sulfates obtenus à partir de mono-, di- ou tristyrylphénol polyalcoxylés dans lesquels le nombre de motifs alcoxylés varie de 2 à 40.

Les tensioactifs anioniques peuvent être sous forme acide (il sont potentiellement anioniques), ou sous une forme partiellement ou totalement salifiée, avec un contre-ion. Le contre-ion peut être un métal alcalin, tel que le sodium ou le potassium, un alcalinoterreux, tel que le calcium, ou encore un ion ammonium de formule N(R)₄⁺ dans laquelle R, identiques ou différents, représentent un atome d'hydrogène ou un radical alkyle en C₁-C₄ éventuellement substitué par un atome d'oxygène.

A titres d'exemples de tensioactifs non ioniques, on peut citer, sans intention de s'y limiter:
- les phénols polyalcoxylés (éthoxylés, propoxylés, éthopropoxylés) substitués par au moins un radical alkyle en C₄-C₂₀, de préférence en C₄-C₁₂, ou substitués par au moins un radical alkylaryle dont la partie alkyle est en C₁-C₆. Plus particulièrement, le nombre total de motifs alcoxylés est compris entre 2 et 100. A titre d'exemple, on peut citer les mono-, di- ou tri (phényléthyl) phénols polyalcoxylés, ou les nonylphénols polyalcoxylés. Parmi les di- ou tristyrylphenols éthoxylés et/ou propoxylés, sulfatés et/ou phosphatés, on peut citer, le di-(phényl-1 éthyl)phénol éthoxylé, contenant 10 motifs oxyéthylénés, le di-(phényl-1 éthyl)phénol éthoxylé, contenant 7 motifs oxyéthylénés, le di-(phényl-1 éthyl)phénol éthoxylé sulfaté, contenant 7 motifs oxyéthylénés, le tri-(phényl-1 éthyl)phénol éthoxylé, contenant 8 motifs oxyéthylénés, le tri-(phényl-1 éthyl)phénol éthoxylé, contenant 16 motifs oxyéthylénés, le tri-(phényl-1 éthyl)phénol éthoxylé sulfaté, contenant 16 motifs oxyéthylénés, le tri-(phényl-1 éthyl)phénol éthoxylé, contenant 20 motifs oxyéthylénés, le tri-(phényl-1 éthyl)phénol éthoxylé phosphaté, contenant 16 motifs oxyéthylénés.
- les alcools ou les acides gras en C₆-C₂₂, polyalcoxylés (éthoxylés, propoxylés, éthopropoxylés). Le nombre des motifs alcoxylés est compris entre 1 et 60. Le terme acide gras éthoxylé inclut aussi bien les produits obtenus par éthoxylation d'un acide gras par l'oxyde d'éthylène que ceux obtenus par estérification d'un acide gras par un polyéthylèneglycol.
- les triglycérides polyalcoxylés (éthoxylés, propoxylés, éthopropoxylés) d'origine végétale ou animale. Ainsi conviennent les triglycérides issus du saindoux, du suif, de l'huile d'arachide, de l'huile de beurre, de l'huile de graine de coton, de l'huile de lin, de l'huile d'olive, de l'huile de palme, de l'huile de pépins de raisin, de l'huile de poisson, de l'huile de soja, de l'huile de ricin, de l'huile de colza, de l'huile de coprah, de l'huile de noix de coco, et comprenant un nombre total de motifs alcoxylés compris entre 1 et 60. Le terme triglycéride éthoxylé vise aussi bien les produits obtenus par éthoxylation d'un triglycéride par l'oxyde d'éthylène que ceux obtenus par trans-estérification d'un triglycéride par un polyéthylèneglycol.
- les esters de sorbitan éventuellement polyalcoxylés (éthoxylés, propoxylés, éthopropoxylés), plus particulièrement les esters de sorbitol cyclisé d'acides gras de C₁₀ à C₂₀ comme l'acide laurique, l'acide stéarique ou l'acide oléique, et comprenant un nombre total de motifs alcoxylés compris entre 2 et 50.

Des émulsifiant utiles sont notamment les produits suivants, tous commercialisés par Rhodia:
- Soprophor TSP/724: tensioactif à base de tristyrylphonol éthopropoxylé
- Soprophor 796/O: tensioactif à base de tristyrylphonol éthopropoxylé
- Soprophor CY 8: tensioactif à base de tristyrylphonol éthoxylé
- Soprophor BSU: tensioactif à base de tristyrylphonol éthoxylé
- Alkamuls RC: tensioactif à base d'huile de ricin éthoxylée
- Alkamuls OR/36: tensioactif à base d'huile de ricin éthoxylée
- Alkamuls T/20: tensioactif à base d'un ester de sorbitan

La formulation comprend avantageusement au moins 4%, de préférence au moins 5%, de préférence au moins 8%, en poids de matière sèche, d'au moins un tensioactif c).

On mentionne que le solvant peut être associé à un tensioactif aromatique et/ou non aromatique.

### Autres détails quant à la formulation phytosanitaire

La formulation phytosanitaire, concentrée, ne comprend pas des quantités importantes d'eau. Typiquement la teneur en eau est inférieure à 50% en poids, avantageusement inférieure à 25% en poids. Elle sera généralement inférieure à 10% en poids.

La formulation est de préférence un formulation liquide, par exemple sous forme d'un concentré emulsifiable (EC), d'une émulsion concentrée (EW) ou d'une micorémulsion (ME). Dans ce cas elle comprend de préférence moins de 500 g/L d'eau, plus préférablement moins de 250 g/L. Elle sera généralement inférieure à 100 g/L.

Les formulations peuvent avantageusement comprendre:
a) de 4 à 60%, de préférence de 10 à 50%, du produit phytosanitaire, en poids de matière active,
b) de 10 à 92%, de préférence de 20 à 80%, du solvant, en poids,
c) de 4 à 60%, de préférence de 5 à 50%, de préférence de 8 à 25%, en poids de matière sèche, d'un émulsifiant, de préférence d'un tensioactif,
d) de 0 à 10% en poids d'eau.

Il n'est pas exclu de réaliser des formulation solides, par exemple des formulations dans lesquelles un liquide comprenant le produit phytosanitaire solubilisé dans le solvant, est supporté par un minéral et/ou dispersé dans une matrice solide.

La formulation peut bien entendu comprendre d'autres ingrédients (ou "autres additifs") que le produit phytosanitaire actif, le(s) solvant(s), le(s) agent(s) émulsifiant(s) optionnel(s) et l'eau optionnelle. Elle peut notamment comprendre des agent de modification de la viscosité, des agents antimousse, notamment des antimousse siliconnés, des agents anti-rebond, des agents anti-lessivage, des charges inertes, notamment des charges minérales, des agents anti-gel...

Notamment les formulations peuvent comprendre des additifs, dits autres additifs, ne rentrant pas dans la définition des produits a), b), ou c), comme:
- d'autres solvants, généralement en faible quantité, par exemple en quantité inférieure à la quantité des solvant b1), b2) et b3), c'est-à-dire en quantité inférieure au solvant du système solvant étant présent dans la plus faible quantité. Un solvant autre n'est pas entendu comme faisant partie du système solvant. A titre d'autres solvants on cite notamment les solvant de la famille des phosphates, phosphonates ou des oxydes de phosphines comme le TEBP, le TBP, le TEPO, le DBBP. On cite également les alkyldiméthyleamides où l'alkyl est en C₆-C₁₈, notamment ceux commercialisés sous la marque Genagen. On cite également les lactates d'esters, notamment ceux commercilisés sous la marque Purasolv. On cite également les esters méthyliques d'acides gras, notamment ceux commercialisés sous la marque Phytorobe. On cite également les diesters de diacides ("DiBasic Esters" en anglais), notamment ceux commercialisé par Rhodia sous les marques Rhodiasolv RPDE, et Rhodiasolv DIB. On cite également les coupes d'hydrocarbures, les amides cycliques, les lactones.
- des inhibiteurs de cristallisation. Il peut s'agir des solvants mentionnés ci-dessus. Il peut également s'agir d'acides gras ou d'alcools gras non polyalkoxylés. On cite par exemple le produit Alkamuls OL700.

Des procédés classiques de préparation de formulations phytosanitaires ou de mélanges de solvants peuvent être mis en oeuvre. On peut opérer par simple mélange des constituants.

La formulation phytosanitaire concentrée est destinée à être répandue sur un champ cultivé ou à cultiver, par exemple un champ de soja, le plus souvent après dilution dans de l'eau, pour obtenir une composition diluée. La dilution est généralement opérée par l'exploitant agricole, directement dans un réservoir ("tank-mix"), par exemple dans le réservoir d'un dispositif destiné à répandre la composition. Il n'est pas exclu que l'exploitant ajoute d'autres produits phytosanitaires, par exemple des fongicides, herbicides, pesticides, insecticides, des fertilisants. Ainsi, la formulation peut être utilisée pour préparer une composition diluée dans l'eau du produit phytosanitaire actif, par mélange d'au moins une part en poids de formulation concentrée avec au moins 10 parts d'eau, de préférence moins de 1000 parts. Les taux de dilution et les quantités à appliquer sur le champ dépendent généralement du produit phytosanitaire et de la dose souhaitable pour traiter le champ; cela peut être déterminé par l'exploitant agricole.

D'autres détails ou avantages pourront apparaître au vu des exemples qui suivent, sans caractère limitatif.

### EXEMPLES

### Exemple 1 - Préparation d'une composition de matière comprenant 80-90% en poids de N,N,N,N-tetraméthyl 2-méthylglutaramide de formule suivante:

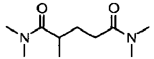

### Etape 1 préliminaire: préparation du mélange d'acides

Produit de départ: "MGN": mélange de dinitriles de composition pondérale suivante :

| | |
|---|---|
| Méthyl-glutaronitrile (MGN): | 84,2% |
| Ethyl-succinonitrile (ESN): | 11 % |
| Adiponitrile (AdN): | 4 % |

Le complément à 100% correspond à différentes impuretés ne comprenant pas des fonctions nitriles.

On mélange du MGN (10,8 g, 0.1 mol) à une solution aqueuse de KOH à 25% en poids (134 g), sous reflux, pendant 19 heures. Le milieu résultant est homogène. On le lave avec t-BuOMe (100 mL), puis on le refroidit à 0°C, puis on l'acidifie avec 50 ml d'une solution aqueuse de HCl à 37% (pH 2). Le diacide obtenu est extrait avec de l'acétate d'éthyle (3 fois 50 ml). Les phases organiques sont combinées et séchées avec MgSO₄, filtrées et évaporées. On obtient 14,4 g de produit solide (température de fusion 76-78°C).

### Etape 2: Conversion en amides

Sous atmosphère d'azote, on charge dans un tricol de 3000 ml à fond arrondi 750 ml de toluène et de la triéthylamine (260 ml, 4.47 mol), et on refroidit à -5°C à l'aide d'un bain de glace et NaCl. On ajoute d'abord de la diéthylamine (500 ml, 7,542 mol, 2,1 équivalent) à cette température, puis 329 g d'un mélange d'acide 2-métylglutarique (84,6% en poids du mélange), d'acide 2-éthylsuccinique (11% en poids du mélange) et d'acide adipique (4,1% en poids du mélange) en solution dans 750 ml de toluène, est ajouté goutte à goutte pendant une heure. Une fois l'addition terminée, on dilue le mélange à l'aide de 300 ml de toluène, laisse le milieu chauffer jusqu'à température ambiante, et on mélange pendant une 12 heures. Ensuite on filtre le sel et on lave avec de l'acétate d'éthyle. Les solutions organiques (issues de la filtration et du lavage) sont combinées et concentrées à pression réduite pour obtenir 378 g de produit brut (huile jaune). On distille (120°C-140°C, 15 Pa) et on obtient 352 g d'une huile jaune qui comprend (analyse par chromatographie en phase gazeuse) 86,3% en poids de N,N,N,N-tetraméthyl 2-méthylglutaramide, 2,2% de N,N,N,N-tetraméthyl-adipamide, et 6,7% de N,N,N,N-tetraméthyl 2-méthylglutaramide.
Le rendement est de 92%.

### Exemple 2 -Préparation d'une composition de matière comprenant plus de 98% en poids de N,N,N,N-tetraméthyl 2-méthylglutaramide de formule suivante:

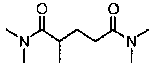

Un mélange d'acide 2-métylglutarique (15 g, 100 mmol) et de 50 ml de chlorure de thionyle est chauffé à 60°C pendant 5 heures. Ensuite l'excès de chlorure de thionyle est éliminé par évaporation sous vide. Le liquide résiduel est dilué dans 100 ml de toluène anhydre, puis la solution résultant est introduite goutte à goutte pendant 45 minutes dans réacteur tricol comprenant de la triéthylamine (28,3 ml, 200 mmol) et de la dimethylamine (20 ml, 1,5 équivalent), à environ 0°C. Ensuite laisse le milieu chauffer jusqu'à température ambiante, et on mélange pendant 6 heures. Ensuite on ajoute 50 ml d'acétate d'éthyle; il en résulte un solide qui est filtré et lavé à l'aide de 50 ml d'acétate d'éthyle. Les solutions organiques (issues de la filtration et du lavage) sont combinées et concentrées pour obtenir 20 g de produit brut (huile jaune). Le produit brut est purifié par distillation sous vide pour obtenir 13,63 g de N,N,N,N-tetraméthyl 2-méthylglutaramide. La pureté est de 98,3%, le rendement est de 67%.

### Exemple 3 - Préparation d'une composition de matière comprenant 80-90 % en poids de N,N,N,N-tetraéthyl 2-méthylglutaramide de formule suivante:

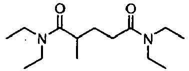

Produit de départ: "MGN": mélange de dinitriles de composition pondérale suivante :

| | |
|---|---|
| Méthyl-glutaronitrile (MGN): | 84,2 % |
| Ethyl-succinonitrile (ESN): | 11 % |
| Adiponitrile (AdN): | 4 % |

Le complément à 100% correspond à différentes impuretés ne comprenant pas des fonctions nitriles.

### Etape 1

On mélange du MGN (10,8 g, 0.1 mol) à une solution aqueuse de KOH à 25% en poids (134 g), sous reflux, pendant 19 heures. Le milieu résultant est homogène. On le lave avec t-BuOMe (100 mL), puis on le refroidit à 0°C, puis on l'acidifie avec 50 ml d'une solution aqueuse de HCl à 37% (pH 2). Le diacide obtenu est extrait avec de l'acétate d'éthyle (3 fois 50 ml). Les phases organiques sont combinées et séchées avec MgSO₄, filtrées et évaporées. On obtient 14,4 g de produit solide (température de fusion 76-78°C).

### Etape 2: Conversion en amides

200 g d'un mélange d'acide 2-métylglutarique (84,6% en poids du mélange), d'acide 2-éthylsuccinique (11% en poids du mélange) et d'acide adipique (4,1% en poids du mélange) et 650 ml de chlorure de thionyl sont chauffés en mélange pendant 5 heures à 60°C. Ensuite l'excès de chlorure de thionyle est éliminé par évaporation sous léger vide. Le liquide résiduel est dilué dans 500 ml de toluène anhydre, puis la solution résultant est introduite goutte à goutte pendant 60 minutes dans réacteur tricol comprenant 500 ml de toluène anhydre, 425 ml de dimethylamine et 400 ml de triéthylamine, à environ 5°C. On mélange pendant 12 heures pour effectuer la réaction. On ajout ensuite 1 L d'acétate d'éthyle et le sel est filtré et lavé à l'aide de 800 ml d'acétate d'éthyle. Le filtrat est partiellement concentré à 500 mL puis lavé avec 1 L d'une solution satirées en NaHCO₃. Après décantation, la couche aqueuse est extraite avec 500 ml d'acétate d'éthyle. Les phases organiques sont combinées et concentrées pour obtenir 279 g de produit brut. On distille à 140°C sous vide poussé et on obtient 205 g de produit final qui est un mélange comprenant principalement (analyse par chromatographie en phase gazeuse) 85,3% en poids de N,N,N,N-tetraéthyl 2-méthylglutaramide, 2,25% de N,N,N,N-tetraéthyl-adipamide, et 5,99% de N,N,N,N-tetraéthyl- 2-éthylsuccinamide.

### Exemple 4 - Préparation d'une composition de matière comprenant plus de 98% en poids de N,N,N,N-tetraéthyl 2-méthylglutaramide de formule suivante:

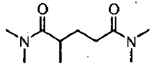

Un mélange d'acide 2-métylglutarique (80 g, 0,542mol) et de 260 ml de chlorure de thionyle (424g, 3,56 mol) est chauffé à 60°C pendant 5 heures. Ensuite l'excès de chlorure de thionyle est éliminé par évaporation sous vide. Le liquide résiduel est dilué dans 200 ml de toluène anhydre, puis la solution résultant est introduite goutte à goutte pendant 60 minutes dans réacteur tricol comprenant 200 ml de toluène, de la triéthylamine (160 ml, 116,2 g, 1,14 mol) et de la diéthylamine (170 ml, 115,6 g, 1,58 mol), à environ 0°C. Ensuite laisse le milieu chauffer jusqu'à température ambiante, et on mélange pendant 6 heures. Après la fin de la réaction (suivie par analyse HPLC), on ajoute 500 ml d'acétate d'éthyle; il en résulte un solide qui est filtré et lavé à l'aide de 250 ml d'acétate d'éthyle. Le filtrat est concentré 500 ml puis lavé avec 500 ml d'une solution aqueuse à 0,2 M de NaOH. Après décantation la phase aqueuse est lavée à l'aide de 250 ml d'acétate d'éthyle. Les phases organiques (issues de la filtration et du lavage) sont combinées et concentrées pour obtenir 110 g de produit brut. On distille à 158°C sous pression de 3 mbar pour obtenir 93,5 g de N,N,N,N-tetraéthyl 2-méthylglutaramide. La pureté est de 98,2%, le rendement est de 66%.

### Exemple 5- Préparation d'une composition de matière comprenant 80-95 % en poids de N,N,N,N-tetra(n-propyl)-2-méthylglutaramide:

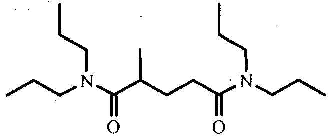

Produit de départ: "MGN": mélange de dinitriles de composition pondérale suivante :

| | |
|---|---|
| Méthyl-glutaronitrile (MGN): | 84,2 % |
| Ethyl-succinonitrile (ESN): | 11 % |
| Adiponitrile (AdN): | 4 % |

Le complément à 100% correspond à différentes impuretés ne comprenant pas des fonctions nitriles.

### Etape 1

On prépare un mélange de diacides comprenant d'acide 2-métylglutarique (84,6% en poids du mélange), d'acide 2-éthylsuccinique (11% en poids du mélange) et d'acide adipique (4,1% en poids du mélange) comme indiqué pour l'étape 1 de l'exemple 3.

### Etape 2

Le mélange de diacides, dévolatilisé (600 g, 4,106 mol) est mélangé à 1210 ml de chlorure de thionyle (1973,5g, 16,423 mol) et chauffé à 60°C pendant 4 heures. Ensuite l'excès de chlorure de thionyle est éliminé par évaporation sous vide pour obtenir 1446g de produit brut. Apres distillation (86/87°C - 350Pa) 1256g de dichlorure sont obtenus (rendement de 83,6% pour cette première étape). Cette étape est répétée une deuxième fois.

Le dichlorure (850g, 4,644 mol) est dilué dans 500 ml de toluène anhydre, puis la solution résultant est introduite goutte à goutte pendant 180 minutes dans réacteur tricol comprenant 4000 ml de toluène, de la triéthylamine (1175 ml, 853,1 g, 11,610 mol) et de la diéthylamine (1780 ml, 1317,2 g, 13,017 mol), à environ 0-10°C. Ensuite laisse le milieu chauffer jusqu'à température ambiante, et on mélange pendant 4 heures. Le milieu est filtre et le solide lave avec de l'acétate d'éthyle. Les filtrats sont combinés et évaporés sous vide. 2000 mL de solution saturée de NaHCO₃ est ajoutée au résidu. Le mélange obtenu est séparé et la phase aqueuse avec de l'acétate d'éthyle (1000mL x 6). Les phases organiques sont combinées, séchées sur sodium sulfate puis évaporées sous vide. 1150g de produit final sont obtenus après distillation sous vide (le rendement est de 78,6% par rapport au dichlorure).

### Exemples 6 à 10 - Formulations phytosanitaires

Par mélange des ingrédients, on prépare des formulations de divers actifs phytosanitaires, de type concentré émulsionnable (EC).

Les formulations comprennent:
- l'actif, en quantité en poids (de matière active) indiquée dans le tableau ci-dessous,
- 10% en poids de tensioactif Alkamuls RC
- et, comme solvant, le reste de composé ou composition de matière des exemples.

Les exemples 11.1 à 11.3 sont des exemples comparatifs où est utilisé comme solvant Rhodiasolv ADMA10, Rhodia (zone Asie Pacifique): Solvant alkyldiméthylamide.

On effectue les tests suivants:
- Observation visuelle à 25°C - On note l'aspect de la formulation et on repère éventuellement la présence de cristaux
- Observation visuelle à 0°C - On place la formulation pendant 7 jours à 0°C et on note l'aspect de la formulation et on repère éventuellement la présence de cristaux (test CIPAC MT39)
- Observation visuelle à 0°C avec nucléation: On introduit un cristal de la matière active dans la formulation ayant passé 7 jours à 0°C pour nucléation, et on place à nouveau la formulation pendant 7 jours à 0°C. On note l'aspect de la formulation et on repère éventuellement la présence de cristaux.

| Exemple | Solvant | Actif | Apparence à 25°C | Apparence à 0°C | Apparence à 0°C avec nucléation |
|---|---|---|---|---|---|
| 6.1 | Exemple 1 | Chlorpyrifos - 40% | Limpide | Limpide | / |
| 6.2 | Exemple 1 | Fastac -10% | Limpide | Limpide | Limpide |
| 6.3 | Exemple 1 | Propanil - 36% | Limpide | Limpide | Limpide |
| 6.4 | Exemple 1 | Tebuconazole - 25% | Limpide | Limpide | Limpide |
| 6.5 | Exemple 1 | Oxyfluorfen - 22% | Limpide | Limpide | Limpide |
| 7.1 | Exemple 2 | Phenmedipham - 16% | Limpide | Limpide | Limpide |
| 7.2 | Exemple 2 | Propanil - 36% | Limpide | Limpide | Limpide |
| 7.3 | Exemple 2 | Tebuconazole - 25% | Limpide | Limpide | Limpide |
| 7.4 | Exemple 2 | Oxyfluorfen - 22% | Limpide | Limpide | Limpide |
| 7.5 | Exemple 2 | Propuxur - 20% | Limpide | Limpide | Limpide |
| 8.1 | Exemple 3 | Tebuconazole - 25% | Limpide | Limpide | Limpide |
| 8.2 | Exemple 3 | Propuxur - 20% | Limpide | Limpide | Limpide |
| 9.1 | Exemple 4 | Phenmedipham - 16% | Limpide | Limpide | Limpide |
| 9.2 | Exemple 4 | Propanil - 36% | Limpide | Limpide | Limpide |
| 9.3 | Exemple 4 | Tebuconazole - 25% | Limpide | Limpide | Limpide |
| 9.4 | Exemple 4 | Oxyfluorfen - 22% | Limpide | Limpide | Limpide |
| 9.5 | Exemple 4 | Propuxur - 20% | Limpide | Limpide | Limpide |
| 10.1 | Exemple 5 | Pendimethalin - 33% | Limpide | Limpide | / |
| 10.2 | Exemple 5 | Triadimenol - 23% | Limpide | Limpide | Limpide |
| 11.1C | Rhodiasolv® ADMA 10 (comparatif) | Oxyfluorfen - 22% | Limpide | Limpide | Cristaux |
| 11.2C | Rhodiasolv® ADMA 10 (comparatif) | Pendimethalin - 33% | Limpide | Cristaux | Cristaux |
| 11.3C | Rhodiasolv® ADMA 10 (comparatif) | Triadimenol - 23% | Limpide | Limpide | Cristaux |

## Revendications

1. Composé de formule (la) suivante :
R²R³NOC-R^{1a}-CONR⁴R⁵ (Ia)
où R², R³, R⁴ et R⁵, identiques ou différents, sont des groupes alkyles linéaires en C₁-C₄,
**caractérisé en ce que** R^{1a} est un groupe divalent de formule (IIa) :
-CH₂-CH₂-(CHR⁶)ₓ- (IIa)
où :
- x est un nombre entier supérieur à 0, et
- R⁶, identique ou différent, est un groupe alkyle en C₁-C₆.

2. Composé selon la revendication 1, **caractérisé en ce que** R², R³, R⁴ et R⁵, identiques ou différents, sont choisis parmi les groupes méthyle, éthyle, n-propyle et n-butyle.

3. Composé selon l'une des revendications précédentes, **caractérisé en ce que** dans le groupe R^{1a} x= 1 et R⁶ = méthyle.

4. Composé selon l'une des revendications précédentes, **caractérisé en ce qu'**il est compris dans une composition de matière, où il représente au moins 10% en poids, de préférence au moins 50% en poids.

5. Composé selon la revendication 4, **caractérisé en ce que** la composition de matière est un produit d'amidification ou de trans-amidification.

6. Composé selon l'une des revendications 4 ou 5, **caractérisé en ce que** la composition de matière comprend en plus un produit de formule (Ib) suivante :
R²R³NOC-R^{1b}-CONR⁴R⁵ (Ib)
où:
- R², R³, -R⁴et R⁵, identiques ou différents, sont tels que définis dans l'une des revendications 1 ou 2,
- R^{1b} est un groupe divalent de formule (IIb):
-CH₂-(CHR⁷)_{z}-(CHR⁶)ₓ-(CHR⁷)_{y}- (IIb)
où:
- x est un nombre entier supérieur à 0,
- y est un nombre entier moyen supérieur ou égal à 0,
- z est un nombre entier moyen supérieur ou égal à 0,
- R⁶, identique ou différent, est un groupe alkyle en C₁-C₆ de préférence en C₁-C₄, et
- R⁷, identique ou différent, est un atome d'hydrogène ou un groupe alkyle en C₁-C₆ de préférence en C₁-C₄.

7. Composé selon la revendication 6, **caractérisé en ce que** dans le groupe R^{1b}:
- x= 1,
- y = z = 0,
- R⁶ = éthyle.

8. Composé selon l'une des revendications 4 à 7, **caractérisé en ce que** la composition de matière est un produit d'amidification ou de trans-amidification d'un mélange d'acide méthylglutarique et d'acide éthylsuccinique, ou d'un diester de ce mélange.

9. Composé selon l'une des revendications 6 ou 7, **caractérisé en ce que** la composition de matière comprend :
- de 70 à 95 % en poids du composé de formule (Ia)
- de 5 à 30 % en poids d'un composé de formule (Ib) et
- éventuellement, au plus 15 % en poids d'un autre composé.

10. Composé selon l'une des revendications précédentes **caractérisé en ce qu'**il présente une température de fusion, le cas échéant dans une composition de matière telle que définie dans l'une des revendications 4 à 9, inférieure ou égale à 25°C.

11. Procédé de préparation d'un composé selon l'une des revendications 1 à 10, le cas échéant dans une composition de matière telle que définie dans l'une des revendications 4 à 9, comprenant une étape d'amidification ou de trans-amidification par un composé de formule R²R³NH et/ou HNR⁴R⁵ d'un composé de formule (l'a) suivante :
R⁸OOC-R^{1a}-COOR⁸ (I'a)
le composé de formule (l'a) étant le cas échéant en mélange avec un composé de formule (I'b) suivante:
R⁸OOC-R^{1b}-COOR⁸ (I'b)
où
- R⁸ est un atome d'hydrogène ou un alkyle en C₁-C₆ de préférence un méthyle, et
- R^{1a} est tel que défini dans l'une des revendications 1 ou 3,
- R^{1b} est tel que défini dans l'une des revendications 6 ou 7.

12. Utilisation du composé de formule (la) tel que défini dans l'une des revendications 1 à 10 comme agent de coalescence, solvant, co-solvant et/ou inhibiteur de cristallisation.

13. Utilisation du composé de formule (la) tel que défini dans l'une des revendications 1 à 10, dans une formulation phytosanitaire, dans une formulation du nettoyage, dans une formulation de décapage, dans une formulation de dégraissage, dans une formulation de lubrifiants ou textiles, dans une formulation de revêtement, dans une formulation de pigments ou encre, dans une formulation plastique.

## Patentansprüche

1. Verbindung der folgenden Formel (la):
R²R³NOC-R^{1a}-CONR⁴R⁵ (Ia)
wobei R², R³, R⁴ und R⁵, identisch oder unterschiedlich, lineare C₁-C₄-Alkylgruppen sind,
**dadurch gekennzeichnet, dass** R^{1a} eine zweiwertige Gruppe der Formel (IIa)
-CH₂-CH₂-(CHR⁶)ₓ- (IIa)
ist, wobei
- x eine ganze Zahl größer als 0 ist, und
- R⁶, identisch oder unterschiedlich, eine C₁-C₆-Alkylgruppe ist.

2. Verbindung nach Anspruch 1, **dadurch gekennzeichnet, dass** R², R³, R⁴ und R⁵, identisch oder unterschiedlich, aus den Methyl-, Ethyl-, n-Propyl- und n-Butylgruppen ausgewählt sind.

3. Verbindung nach einem der vorangehenden Ansprüche, **dadurch gekennzeichnet, dass** in der Gruppe R^{1a} x = 1 und R⁶ = Methyl ist.

4. Verbindung nach einem der vorangehenden Ansprüche, **dadurch gekennzeichnet, dass** sie in einer Materialzusammensetzung umfasst ist, wobei sie mindestens 10 Gew.-%, vorzugsweise mindestens 50 Gew.-%, darstellt.

5. Verbindung nach Anspruch 4, **dadurch gekennzeichnet, dass** die Materialzusammensetzung ein Amidierungs- oder ein Trans-Amidierungsprodukt ist.

6. Verbindung nach einem der Ansprüche 4 oder 5, **dadurch gekennzeichnet, dass** die Materialzusammensetzung zusätzlich ein Produkt der folgenden Formel (Ib) umfasst:
R²R³NOC-R^{1b}-CONR⁴R⁵ (Ib)
wobei:
- R², R³, R⁴ und R⁵, identisch oder unterschiedlich, nach einem der Ansprüche 1 oder 2 sind,
- R^{1b} eine zweiwertige Gruppe der Formel (IIb)
-CH₂-(CH R⁷)_{Z}-(CHR⁶)_{X}-(CH R⁷)_{y}- (IIb)
ist, wobei:
- x eine ganze Zahl größer als 0 ist,
- y eine mittlere ganze Zahl größer oder gleich 0 ist,
- z eine mittlere ganze Zahl größer oder gleich 0 ist
- R⁶, identisch oder unterschiedlich, eine C₁-C₆-, vorzugsweise eine C₁-C₄-Alkylgruppe ist, und
- R⁷, identisch oder unterschiedlich, ein Wasserstoffatom oder eine C₁-C₆-, vorzugsweise eine C₁-C₄-Alkylgruppe, ist.

7. Verbindung nach Anspruch 6, **dadurch gekennzeichnet, dass** in der Gruppe R^{1b}:
- x= 1 ,
- y = z = 0,
- R⁶ = Ethyl.

8. Verbindung nach einem der Ansprüche 4 bis 7, **dadurch gekennzeichnet, dass** die Materialzusammensetzung ein Amidierungs- oder Trans-Amidierungsprodukt eines Gemischs aus Methylglutarsäure und Ethylbernsteinsäure oder eines Diesters dieses Gemischs ist.

9. Verbindung nach einem der Ansprüche 6 oder 7, **dadurch gekennzeichnet, dass** die Materialzusammensetzung umfasst:
- 70 bis 95 Gew.-% der Verbindung der Formel (la),
- 5 bis 30 Gew.-% einer Verbindung der Formel (Ib) und
- eventuell höchstens 15 Gew.-% einer anderen Verbindung.

10. Verbindung nach einem der vorangehenden Ansprüche, **dadurch gekennzeichnet, dass** sie eine Schmelztemperatur, gegebenenfalls in einer Materialzusammensetzung nach einem der Ansprüche 4 bis 9, von unter oder gleich 25 °C aufweist.

11. Herstellungsverfahren einer Verbindung nach einem der Ansprüche 1 bis 10, gegebenenfalls in einer Materialzusammensetzung nach einem der Ansprüche 4 bis 9, das einen Amidierungs- oder Trans-Amidierungsschritt durch eine Verbindung der Formel R²R³NH und/oder HNR⁴R⁵ einer Verbindung der folgenden Formel (I'a)
R⁸OOC-R^{1a}-COOR⁸ (I'a)
umfasst, wobei die Verbindung der Formel (I'a) gegebenenfalls gemischt mit einer Verbindung der folgenden Formel (I'b)
R⁸OOC-R^{1b}-COOR⁸ (I'b)
ist, wobei:
- R⁸ ein Wasserstoffatom oder ein C₁-C₆-Alkyl, vorzugsweise ein Methyl, ist, und
- R^{1a} nach einem der Ansprüche 1 oder 3 ist,
- R^{1b} nach einem der Ansprüche 6 oder 7 ist.

12. Verwendung der Verbindung der Formel (la) nach einem der Ansprüche 1 bis 10 als Koaleszenzmittel, Lösungsmittel, Ko-Lösungsmittel und/oder Kristallisationshemmer.

13. Verwendung der Verbindung der Formel (la) nach einem der Ansprüche 1 bis 10 in einer Pflanzenschutzformulierung, ein einer Reinigungsformulierung, in einer Beizformulierung, in einer Entfettungsformulierung, in einer Schmier- oder Textilformulierung, in einer Beschichtungsformulierung, in einer Pigment- oder Tintenformulierung, in einer Kunststoffformulierung.

## Claims

1. A compound of the following formula:
R²R³NOC-R^{1a}-CONR⁴R⁵ (Ia)
wherein R², R³, R⁴ and R⁵, either identical or different, are linear C₁-C₄ alkyl groups, **characterized in that** R^{1a} is a divalent group of formula (IIa):
-CH₂-CH₂-(CHR⁶)ₓ- (IIa)
wherein:
- x is an integer greater than 0, and
- R⁶, either identical or different, is a C₁-C₆ alkyl group.

2. The compound according to claim 1, **characterized in that** R², R³, R⁴ and R⁵, either identical or different, are selected from among methyl, ethyl, n-propyl, and n-butyl groups.

3. The compound according to one of the preceding claims, **characterized in that** in the group R^{1a} x=1 and R⁶ = methyl.

4. The compound according to one of the preceding claims, **characterized in that** it is comprised in a material composition, wherein it represents at least 10% by weight, preferably at least 50% by weight.

5. The compound according to claim 4, **characterized in that** the material composition is an amidification or trans-amidification product.

6. The compound according to one of claims 4 or 5, **characterized in that** the material composition further comprises a product of the following formula (Ib):
R²R³NOC-R^{1b}-CONR⁴R⁵ (Ib)
wherein:
- R², R³, R⁴ and R⁵, either identical or different, are as defined in one of claims 1 or 2,
- R^{1b} is a divalent group of formula (IIb):
-CH₂-(CHR⁷)_{z}-(CHR⁶)ₓ-(CHR⁷)_{y}- (IIb)
wherein:
- x is an integer greater than 0,
- y is an average integer greater than or equal to 0,
- z is an average integer greater than or equal to 0,
- R⁶, either identical or different, is a C₁-C₆, preferably C₁-C₄, alkyl group, and
- R⁷, either identical or different, is a hydrogen atom or a C₁-C₆, preferably C₁-C₄, alkyl group.

7. The compound according to claim 6, **characterized in that** in the R^{1b} group:
- x = 1,
- y = z = 0,
- R⁶ = ethyl.

8. The compound according to one of claims 4 to 7, **characterized in that** the material composition is a product from amidification or trans-amidification of a mixture of methylglutaric acid and of ethylsuccinic acid, or of a diester of this mixture.

9. The compound according to one of claims 6 or 7, **characterized in that** the material composition comprises:
- from 70 to 95% by weight of the compound of formula (Ia)
- from 5 to 30% by weight of a compound of formula (Ib) and
- optionally, at most 15% by weight of another compound.

10. The compound according to one of the preceding claims, **characterized in that** it has a melting temperature, if necessary, in a material composition as defined in one of claims 4 to 9, of less than or equal to 25°C.

11. A method for preparing a compound according to one of claims 1 to 10, if necessary, in a material composition as defined in one of claims 4 to 9, comprising a amidification or trans-amidification step with a compound of formula R²R³NH and/or HNR⁴R⁵ product for amidification or trans-amidification of a compound of the following formula (I'a):
R⁸OOC-R^{1a}-COOR⁸ (I'a)
the compound of formula (I'a) if necessary being in a mixture with a compound of the following formula (I'b):
R⁸OOC-R^{1b}-COOR⁸ (I'b)
wherein
- R⁸ is a hydrogen atom or a C₁-C₆ alkyl, preferably methyl, group,
- R^{1a} is as defined in one of claims 1 or 3,
- R^{1b} is as defined in one of claims 6 or 7.

12. The use of the compound of formula (Ia) as defined in one of claims 1 to 10 as a coalescence agent, solvent, co-solvent and/or crystallization inhibitor.

13. The use of the compound of formula (Ia) as defined in one of claims 1 to 10, in a phytosanitary formulation, in a cleaning formulation, in a pickling formulation, in a degreasing formulation, in a formulation of lubricants or textiles, in a coating formulation, in a formulation of pigments or ink, a formulation of plastics.
